# FASCICULE DE BREVET EUROPEEN

(11) **EP 1 353 641 B1**
(45) Date de publication et mention de la délivrance du brevet: **29.03.2006**
(21) Numéro de dépôt: 02710086.6
(22) Date de dépôt: 08.01.2002
(51) Int. Cl.: A61K 8/06, A61K 8/27, A61K 8/29, A61K 8/81, A61K 8/891, A61K 8/91, A61Q 17/04

(54) **COMPOSITION COSMETIQUE CONTENANT DES FILTRES MINERAUX**
KOSMETISCHE ZUBEREITUNG ENTHALTEND MINERALE FILTER
COSMETIC COMPOSITION CONTAINING MINERAL FILTERS

(30) Priorité: 15.01.2001 FR 0100484
(43) Date de publication de la demande: 22.10.2003
(73) Titulaire: L'OREAL, 75008 Paris (FR)
(72) Inventeur: LENNON, Paula, F-69003 Lyon (FR); LORANT, Raluca, F-94320 Thiais (FR)
(74) Mandataire: Miszputen, Laurent
(86) Numéro de dépôt international: PCT/FR2002/000046
(87) Numéro de publication internationale: WO 2002/055046

(56) Documents cités:
- EP-A1- 0 748 624
- EP-A1- 1 034 779
- EP-A1- 1 172 089
- EP-A2- 1 210 929
- US-A- 2 283 214
- US-A- 4 369 123
- US-A- 5 744 126
- US-A- 5 955 003
- US-A- 6 066 326

## Description

La présente invention se rapporte à une composition sous forme d'émulsion comprenant un oxyde minéral et un oligomère ou un polymère dérivé de polyoléfine, notamment sous la forme d'une émulsion eau-dans-huile, et à l'utilisation de ladite composition, notamment dans le domaine cosmétique, en particulier pour le soin et/ou la protection solaire de la peau, des lèvres et des cheveux.

Dans le domaine cosmétique, il est courant d'utiliser des filtres chimiques pour obtenir des produits de protection solaire. Ces filtres chimiques peuvent être introduits assez facilement dans les émulsions par dispersion dans la phase huileuse ou aqueuse de l'émulsion, selon leur caractère lipophile ou hydrophile.

Pour obtenir des forts indices de protection, il est nécessaire d'augmenter le taux de filtres chimiques. Toutefois, pour des raisons de tolérance, on cherche à éviter d'utiliser un taux trop important de filtres chimiques et on préfère introduire à côté ou à la place des filtres chimiques, des filtres physiques minéraux, en particulier des oxydes métalliques comme par exemple le dioxyde de titane et l'oxyde de zinc qui confèrent d'excellentes propriétés protectrices contre les U.V. et une très bonne tolérance cutanée.

Toutefois, l'introduction de ces oxydes métalliques pose des problèmes d'agrément cosmétique. En effet, les produits de protection solaire les contenant se présentent souvent sous forme d'émulsions plus ou moins épaisses, difficiles à appliquer et à étaler, lourdes et poisseuses. En outre, avec certains filtres minéraux comme le dioxyde de titane, à ces défauts s'ajoute un effet de blanchissement lors de l'étalement sur la peau.

Par ailleurs, on cherche à obtenir des émulsions de protection solaire qui présentent une texture fluide car la texture fluide les rend plus pratiques, plus faciles à appliquer et plus agréables à utiliser. Toutefois, les émulsions fluides sont aussi plus difficiles à réaliser avec des filtres minéraux car les oxydes métalliques présentent l'inconvénient de provoquer la déstabilisation des émulsions dans lesquelles on veut les introduire, et notamment quand il s'agit d'émutsions très fluides. Et cette difficulté d'introduction d'oxydes métalliques est encore plus grande lorsque le taux d'oxydes dépasse 1% de la composition finale.

Les phénomènes d'instabilité se traduisent en particulier par l'agglomération des particules solides, le crémage et la sédimentation des émulsions, un aspect hétérogène des émulsions, et une évolution de la texture dans le temps, cette évolution se traduisant par un épaississement de la texture qui devient, en outre, granuleuse et hétérogène.

Le but de la présente invention est de surmonter ces inconvénients et de proposer une composition permettant d'obtenir des émulsions stables même si elles sont très fluides, présentant de forts Indices de protection grâce à la présence de filtres minéraux et ayant également un grand agrément cosmétique.

La demanderesse a trouvé de manière surprenante que l'utilisation d'un oligomère ou polymère dérivé de polyoléfine, comportant au moins une partie polaire, permettait d'atteindre ce but et notamment d'obtenir une émulsion contenant des oxydes métalliques, ayant de bonnes propriétés cosmétiques (toucher léger, frais et riche à la fois) et une bonne stabilité dans le temps, même si l'émulsion est très fluide et même si elle contient une proportion importante d'oxydes métalliques.

Aussi, la présente invention a pour objet une composition sous forme d'émulsion, contenant dans un milieu physiologiquement acceptable, au moins une phase aqueuse, au moins une phase huileuse, au moins un oxyde métallique et au moins un oligomère ou un polymère dérivé de polyoléfine, comportant au moins une partie polaire.

La présente invention a aussi pour objet l'utilisation d'au moins un oligomère ou un polymère dérivé de polyoléfine, comportant au moins une partie polaire, pour stabiliser une émulsion contenant au moins un oxyde métallique.

On entend dans la présente demande par « milieu physiologiquement acceptable » un milieu compatible avec la peau y compris le cuir chevelu, les muqueuses, les yeux et/ou les cheveux.

On entend dans la présente demande « composition sous forme d'émulsion » aussi bien les émulsions huile-dans-eau (H/E) comportant une phase huileuse dispersée dans une phase aqueuse que les émulsions eau-dans-huile (E/H) comportant une phase aqueuse dispersée dans une phase huileuse et les émulsions multiples et par exemple les émulsions triples (E/H/E ou H/E/H). Selon un mode préféré de réalisation de l'invention, la composition est une émulsion E/H.

Les compositions obtenues selon l'invention présentent l'avantage de s'étaler facilement et d'être absorbées rapidement et complètement dans la peau. En outre, quand elles contiennent du dioxyde de titane, elles présentent l'avantage de ne pas donner d'effet blanchissant lors de l'application sur la peau.

Par ailleurs, les compositions selon l'invention peuvent contenir un pourcentage important de filtres physiques et donc donner un SPF (facteur de protection solaire) élevé tout en étant parfaitement stables et agréables à utiliser.

Les oligomères et polymères utilisables dans l'invention sont connus dans d'autres domaines. Ainsi, ils sont décrits par exemple dans les documents US-A-5,129,972 et US-A-4,919,179, comme stabilisants d'émulsions explosives. Par ailleurs, ces composés sont connus comme stabilisants de compositions fertilisantes (voir les documents US-A-5,518,517 et US-A-5,858,055) en vue d'obtenir un relargage contrôlé des substances fertilisantes. Les oligomères et polymères sont également connus dans la demande EP1210929 comme agents émulsionnants dans des compositions cosmétiques ou pharmaceutiques

L'utilisation d'oligomères ou de polymères dérivés de polyoléfines comme émulsionnants dans la composition de l'invention contenant des oxydes métalliques permet d'obtenir des émulsions stables même si elles sont très fluides et ont donc une faible viscosité.

Les oligomères et polymères utilisés dans la composition de l'invention sont constitués d'une partie apolaire polyoléfinique et d'au moins une partie polaire. Ils peuvent présenter une structure de type bloc ou peigne.

La partie apolaire polyoléfinique comprend au moins 40 atomes de carbone et de préférence de 60 à 700 atomes de carbone. Cette partie apolaire peut être choisie parmi les polyoléfines telles que les oligomères, les polymères et/ou les copolymères d'éthylène, d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne. Ces polyoléfines sont hydrogénées ou non.

Par ailleurs, les oligomères ou polymères dérivés de polyoléfine utilisés dans la composition de l'invention comportent au moins une partie polaire. Cette partie polaire confère aux dérivés de polyoléfines des propriétés amphiphiles. Ainsi, ces oligomères ou polymères abaissent la tension interfaciale (eau / huile) d'au moins 10 mN/m quand ils sont présents à une concentration de 0,01 % en poids par rapport au poids total de la phase huileuse. Par exemple, la polyoléfine à terminaison succinique commercialisée sous la dénomination Lubrizol 2724 par la société Lubrizol, à une concentration de 0,01 % en poids par rapport au poids total de la phase huileuse, abaisse la tension interfaciale de 15 mN/m à l'interface d'une phase aqueuse constituée d'une solution aqueuse à 1% de MgSO4, et d'une phase huileuse comportant un mélange d'huiles (isohexadécane /polyisobutène hydrogéné/silicone volatile dans un rapport 8/6/4).

La partie polaire des oligomères ou polymères de l'invention peut être anionique, cationique, non ionique, zwitterionique ou amphotère. Elle est par exemple constituée de polyalkylène glycols ou de polyalkylène imines, ou encore d'acides ou de diacides carboxyliques, de leurs anhydrides ou de leurs dérivés, et leurs mélanges. Les oligomères ou polymères à partie polaire acide carboxylique peuvent être par exemple issus de la réaction entre, une polyoléfine et au moins un acide ou anhydride carboxylique choisi dans le groupe comprenant l'acide maléique, l'anhydride maléique, l'acide fumarique, l'acide itaconique, l'acide citraconique, l'acide mésaconique, l'acide aconitique. De préférence, la partie polaire est constituée par l'acide ou l'anhydride succinique, leurs dérivés esters ou amides, les sels d'ions alcalins, alcalino-terreux ou organiques correspondants, ou bien encore par du polyoxyéthylène.

Les oligomères ou polymères dérivés de polyoxyéthylène peuvent être par exemple choisis parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p.1582-1586).

Les oligomères ou polymères dérivés d'acide ou d'anhydride succinique peuvent être choisis notamment parmi les dérivés polyoléfines d'acide ou d'anhydride succinique décrits dans les brevets US-A-4,234,435, US-A-4,708,753, US-A-5,129,972, US-A-4,931, 110, GB-A-2,156,799 et US-A-4,919,179 La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatique, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753.

Comme polyoléfines à terminaison succinique, on peut citer notamment les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol.

Un autre exemple de tensioactif polymérique utilisable dans l'invention est le produit de la réaction de l'anhydride maléique avec le polyisobutylène, tel que le produit commercialisé sous la dénomination Glissopal SA par la société BASF.

La quantité d'oligomère(s) ou de polymère(s) dans la composition de l'invention peut aller par exemple de 0,1 à 20 % en poids de matière active, de préférence de 0,2 à 10 % en poids et mieux de 0,5 à 5 % en poids par rapport au poids total de la composition. On peut utiliser un ou plusieurs oligomères ou polymères dérivés de polyoléfines.

Les oxydes métalliques utilisables dans le cadre de la présente invention sont tous ceux déjà connus en soi pour leur activité photoprotectrice. Ainsi, ils peuvent être notamment choisis parmi les oxydes de titane (dioxyde de titane amorphe ou cristallisé sous forme rutile et/ou anatase), de zinc, de fer, de zirconium, de cérium ou leurs mélanges.

Ces oxydes métalliques sont sous forme de particules ayant une taille nanométrique (nano-pigments). Sous forme de nano-pigments, les tailles moyennes des particules vont par exemple de 5 à 100 nm. On utilise de préférence dans la composition de l'invention des nano-pigments.

Par ailleurs, les oxydes métalliques peuvent être enrobés ou non. En particulier, les oxydes métalliques peuvent comporter un enrobage hydrophobe. On entend ici par « enrobage hydrophobe » un enrobage n'ayant pas d'affinité avec l'eau et ne se laissant pas mouillé par l'eau. Cet enrobage est obtenu par un ou plusieurs traitements de surface de l'oxyde métallique par un ou plusieurs composés hydrophobes.

Les oxydes métalliques enrobés et notamment à enrobage hydrophobe, utilisés selon l'invention peuvent par exemple avoir subi un ou plusieurs traitements avec un ou plusieurs composés choisis parmi l'alumine, la silice, les dérivés de l'aluminium (par exemple stéarate et laurate), les composés du silicium (par exemple silicones, polydiméthylsiloxanes, alcoxysilanes, siloxy-silicates), les composés du sodium, les oxydes de fer, les esters de fer (par exemple stéarate), les acides gras, les alcools gras et leurs dérivés (tels que l'acide stéarique, l'acide hydroxystéarique, l'alcool stéarylique, l'alcool hydroxystéarylique, l'acide laurique et leurs dérivés), la lécithine, les cires (par exemple la cire de camauba), les polymères (méth)acryliques (par exemple les polyméthylmethacrylates), les composés fluorés (par exemple les composés perfluoroalkyle et les perfluoroalkyle éthers). Les oxydes peuvent aussi être traités par un mélange de ces composés ou ils peuvent comprendre plusieurs de ces enrobages successifs.

De manière préférée, les oxydes métalliques utilisés dans la composition de l'invention sont choisis parmi les oxydes de titane de préférence enrobés, les oxydes de zinc de préférence enrobés, et leurs mélanges.

Les oxydes de titane non enrobés peuvent par exemple être ceux vendus par la société Tayca sous les dénominations commerciales Microtitanium Dioxide MT 500 B ou Microtitanium Dioxide MT 600 B ; ceux vendus par la société Tioxide sous la dénomination commerciale Selected Tioxide A-HRC ou ceux vendus par la société Rhodia Chimie sous la dénomination commerciale Mirasun TW60 (dispersion à 40% dans l'eau).

Les oxydes métalliques à enrobage hydrophobe susceptibles d'être utilisés dans la composition de l'invention peuvent être choisis notamment parmi les oxydes et nano-oxydes de titane traités par :
- l'alumine et l'acide stéarique, comme le produit commercialisé sous la dénomination UV-Titan M160 par la société Kemira, et le produit commercialisé sous la dénomination ST-430C par la société Inanata ;
- les polydiméthylsiloxanes (PDMS), comme les produits commercialisés sous la dénomination Eusolex T-2000 par la société Merck, sous la dénomination UV Titan X170 par la société Eckart, sous la dénomination Si-UFTR-Z par la société Myoshi ;
- les alcoxysilanes, comme le produit commercialisé sous la dénomination Covascreen T1 par la société Wackherr ;
- les composés perfluoroalkyle, comme le produit commercialisé sous la dénomination PF-7 TiO2 MT-600B par la société Daito,
- les perfluoroalkyl éthers, comme le produit commercialisé sous la dénomination TiO2 VF-25-3A par la société Toshiki ;
- les siloxy-silicates, par exemple comme le produit commercialisé sous la dénomination TSS-1 par la société ISK ;
- les polyméthylméthacrylates, comme par exemple le produit commercialisé sous la dénomination PW COVASIL S par la société Wackherr ;
- la lécithine, comme le produit commercialisé sous la dénomination DUOTERC CW 5-25 par la société Sachtieben ;
- la cire de carnauba, comme le produit commercialisé sous la dénomination UVT-PT 951101 par la société Kemira ;
- la silice et l'alumine, comme les produits commercialisés sous les dénominations Microtitanium Dioxide MT 500 SA et Microtitanium Dioxide MT 100 SA par la société Tayca, et Tioveil Fin, Tioveil OP, Tioveil MOTG et Tioveil IPM par la société Uniqema ;
- l'alumine et le stéarate d'aluminium, comme le produit commercialisé sous la dénomination Microtitanium Dioxide MT 100 T par la société Tayca ;
- l'alumine et le laurate d'aluminium, comme le produit commercialisé sous la dénomination Microtitanium Dioxide MT 100 S par la société Tayca ;
- des oxydes de fer et le stéarate de fer, comme le produit commercialisé sous la dénomination Microtitanium Dioxide MT 100 F par la société Tayca ;
- la silice, l'alumine et la silicone, comme les produits commercialisés sous les dénominations Microtitanium Dioxide MT 100 SAS, Microtitanium Dioxide MT 600 SAS et Microtitanium Dioxide MT 500 SAS par la société Tayca ;
- l'octyltriméthoxysilane, comme le produit commercialisé sous la dénomination T-805 par la société Degussa ;
- l'alumine et la glycérine, comme le produit commercialisé sous la dénomination UVT-M212 par la société Kemira ;
- l'alumine et la silicone, comme le produit commercialisé sous la dénomination UVT-M262 par de la société Kemira.

Ces oxydes et nano-oxydes de titane enrobés hydrophobes peuvent se présenter sous forme de charge solide ou sous forme de dispersion dans un milieu huileux. Comme dispersions d'oxyde de titane enrobé, on peut citer par exemple les produits indiqués ci-dessus, commercialisés par la société Uniqema sous les dénominations TIOVEIL FIN (nano-oxyde de titane dispersé dans benzoate d'alcool C12-C15 avec, comme dispersant, un polycondensat d'acide hydrostéarique) et TIOVEIL MOTG (nano-oxyde de titane dispersé dans huile minérale/triglycérides avec, comme dispersant, un polycondensat d'acide hydroxystéarique) ; le produit commercialisé sous la dénomination COVASCREEN TI par la société Wackherr (dispersion huileuse de TiO2 enrobé par du triméthoxy-octylsilane à 60 %) ; le produit commercialisé sous la dénomination DAITOPERSION TI-30 par la société Daito (dispersion de nano-oxyde de titane enrobé par du polyméthylhydrogènesiloxane dans cyclométhicone et diméthicone copolyol) ; le produit commercialisé sous la dénomination TIOSPERSE ULTRA par la société Collaborative Laboratories (nano-oxyde de titane enrobé par acide stéarique/alumine, dispersé dans du benzoate d'hydroxystéarate d'éthyl-2-hexyle) ; le produit commercialisé sous la dénomination MIBRID DISPERSION SAS4 5050 par la société Myoshi (nano-oxyde de titane enrobé par alumine/acide stéarique puis par polydiméthylsiloxane, dispersé dans cyclopentasiloxane) ; le produit commercialisé sous la dénomination SPD-T1 par la société Shin-Etsu (nano-oxyde de titane enrobé par un polymère acrylique greffé silicone et dispersé dans le cyclopentadiméthylsiloxane).

Selon un mode préféré de réalisation de l'invention, on utilise dans la composition de l'invention les oxydes de titane suivants : Sl-UFTR-Z, TIOVEIL MOTG, UV-TITAN M160, EUSOLEX T-2000, PF-7 T102 MT-600B, PW COVASIL S, TSS-1, COVASCREEN TI, DAITOPERSION TI-30, TIOSPERSE ULTRA, UV-TITAN X 170, MIBRID DISPERSION SAS4 5050 ,SPD-T1, TIOVEII, FIN, et plus préférentiellement les produits UV-TITAN X 170, TIOSPERSE ULTRA, PW COVASIL S, MIBRID DISPERSION SAS4 5050, SPD-T1.

Comme oxydes de zinc utilisables dans la composition de l'invention, on peut citer par exemple :
- les nano-oxydes de zinc non enrobés, tels que ceux commercialisés sous la dénomination Z-cote par la société Sunsmart; ceux commercialisés sous la dénomination Nanox par la société Elementis ; ceux commercialisés sous la dénomination Nanogard WCD 2025 par la société Nanophase Technologies ;
- les nano-oxydes de zinc enrobés, comme par exemple ceux commercialisés sous la dénomination Oxide zinc CS-5 par la société Toshibi (ZnO enrobé par polymethylhydrogenesiloxane); ceux commercialisés sous la dénomination Nanogard Zinc Oxide FN par la société Nanophase Technologies (en dispersion à 40% dans le Finsolv TN, benzoate d'alcools en C12-C15) ; ceux commercialisés sous la dénomination DAITOPERSION ZN-30 et Zn-50 par la société Daito (dispersions dans cyclopolyméthylsiloxane / polydiméthylsiloxane oxyéthyléné, contenant 30% ou 50% de nano-oxydes de zinc enrobés par la silice et la polyméthylthydrogènesiloxane); ceux commercialisés sous la dénomination NFD Ultrafine ZnO par la société Daikin (ZnO enrobé par phosphate de perfluoroalkyle et copolymère à base de perfluoroalkyléthyle en dispersion dans du cyclopentasiloxane) ; ceux commercialisés sous la dénomination SPD-Z1 par la société Shin-Etsu (ZnO enrobé par polymère acrylique greffé silicone, dispersé dans cyclodiméthylsiloxane) ; ceux commercialisés sous la dénomination Escalol Z100 par la société ISP (ZnO traité alumine et dispersé dans le mélange methoxycinnamate d'ethylhexyle / copolymère PVP-hexadecene / methicone) ; ceux commercialisés sous la dénomination Fuji ZnO-SMS-10 par la société Fuji Pigment (ZnO enrobé silice et polymethylsilsesquioxane) ; ceux commercialisés sous la dénomination Nanox Gel TN par la société Elementis (ZnO dispersé à 55% dans du benzoate d'alcools en C12-C15 avec polycondensat d'acide hydroxystéarique).

On peut utiliser aussi un mélange de ces différents oxydes métalliques enrobés ou non enrobés dans la composition de l'invention.

La quantité d'oxydes métalliques dans la composition de l'invention peut aller par exemple de 0,5 à 30 % en poids de matière active, de préférence de 2 à 20 % en poids de matière active et mieux de 7 à 15 % en poids de matière active par rapport au poids total de la composition.

La composition de l'invention est destinée à une application topique et plus particulièrement à une application sur la peau, les cheveux et/ou les muqueuses.

Elle peut constituer notamment une composition cosmétique et/ou dermatologique.

La viscosité de la composition selon l'invention peut varier dans une large mesuré selon le type de produit désiré et peut aller d'un lait très fluide à une crème compacte. Généralement, cette viscosité mesurée à la température ambiante (environ 25°C) avec un viscosimètre Mettler RM 180 (Rheomat) va de 60 à 3000 cP (centipoises) (60 à 3000 mPa.s) et de préférence de 80 à 2500 cP (80 à 2500 mPa.s).

Selon un mode préféré de réalisation de l'invention, la composition se présente sous forme d'une émulsion E/H, notamment sous forme d'une émulsion fluide, c'est-à-dire présentant une viscosité allant de 60 à 600 cP (60 à 600 mPa.s) et encore mieux de 80 et 250 cP (80 à 250 mPa.s), la viscosité étant mesurée au viscosimètre Mettler RM 180 (Rheomat) avec un mobile M2, à 25°C et à une vitesse de 200 tours /min. Par exemple, la valeur de la viscosité mesurée au viscosimètre Mettier RM 180 (Rheomat) avec un mobile M2, à 25°C et à une vitesse de 200 tours /min, peut ainsi aller de 20 à 40 unités de déviation ce qui correspond à une viscosité allant de 100 à 250 cps (100 à 250 mpa.s). On obtient ainsi une émulsion assez fluide, très agréable à utiliser car elle s'étale facilement et de façon homogène sans laisser de sensation de gras ou de film rêche ou collant.

La composition selon l'invention comprend une phase huileuse, qui peut être constituée par tous les corps gras et notamment les huiles, classiquement utilisés dans le domaine cosmétique. Selon un mode préféré de réalisation de l'invention, la composition comprend au moins une huile.

Parmi les huiles utilisables dans la composition de l'invention, on peut citer par exemple les huiles végétales telles que l'huile de noyaux d'abricot et l'huile de soja ; les huiles minérales comme l'huile de vaseline ; les huiles de synthèse comme l'isohexadécane et le cyclohexadécane ; les huiles de silicone volatiles ou non volatiles et les huiles fluorées. Comme huiles de silicone volatiles, on peut citer notamment les polydiméthylsiloxanes cycliques ou cyclométhicones qui comportent d'environ 3 à 9 atomes de silicium et de préférence de 4 à 6 atomes de silicium, telles que le cyclohexadiméthyisiloxane (ou cyclohexaméthicone) et le cyclopentadiméthyl-siloxane (ou cyclopentaméthicone), et les polydiméthylsiloxanes linéaires volatiles comportant d'environ 3 à 9 atomes de silicium. Selon un mode particulier de réalisation de l'invention, la composition comprend au moins une huile de silicone.

Les autres corps gras susceptibles d'être présents dans la phase huileuse peuvent être par exemple les acides gras, les alcools gras et les cires telles que la vaseline ou la cire d'abeille.

La quantité de phase huileuse dans la composition de l'invention peut aller par exemple de 5 à 80 % et de préférence de 40 à 70 % en poids par rapport au poids total de l'émulsion. La phase aqueuse de l'émulsion peut représenter par exemple de 50 à 95 % et de préférence de 60 à 90 % en poids par rapport au poids total de l'émulsion.

De façon connue, la composition de l'invention peut contenir également des adjuvants habituels dans le domaine cosmétique, tels que les actifs, les conservateurs, les antioxydants, les agents complexants, les solvants, les parfums, les charges, les filtres solaires, les bactéricides, les électrolytes (tels que sulfate de magnésium), les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent 1 être introduits dans la phase huileuse, dans la phase aqueuse et/ou dans les vésicules lipidiques. Ces adjuvants ainsi que leurs concentrations doivent être tels qu'ils ne modifient pas la propriété recherchée pour la composition de l'invention.

Selon un mode préféré de réalisation de l'invention, la composition comprend au moins un filtre solaire. Comme filtres solaires, la composition de l'invention peut comprendre tous les filtres UVA et UVB utilisables dans le domaine cosmétique.

Comme filtres UVB, on peut citer par exemple:
(1) les dérivés de l'acide salicylique, en particulier le salicylate d'homomenthyle et le salicylate d'octyle;
(2) les dérivés de l'acide cinnamique, en particulier le p-méthoxycinnamate de 2-éthylhexyle, commercialisé par la société Givaudan sous la dénomination Parsol MCX ;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoique ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple:
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) les filtres actifs dans l'UV-A de formule (IV) suivante : 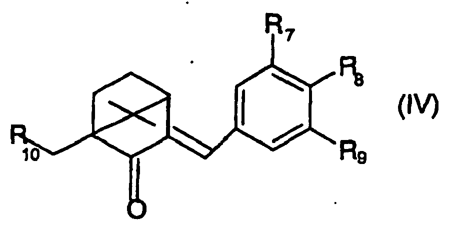 dans laquelle :
   - R₇ et R₉, identiques ou différents, désignent un hydrogène, un halogène, un radical OH, un radical alkyle, saturé ou non, linéaire ou ramifié en C₁-C₁₀ ; un radical alcoxy, saturé ou non, linéaire ou ramifié en C₁-C₁₀ ou un groupe HSO₃ ;
   - R₁₀ désigne un hydrogène ou HSO₃ ;
   - R₈ désigne un groupe OH ; un groupe OR₁₁, où R₁₁ désigne un radical alkyle, saturé ou insaturé, linéaire ou ramifié en C₁-C₁₀; ou bien un groupe de structure suivante : 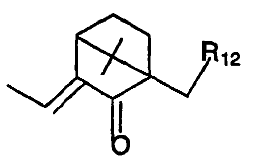
   dans laquelle R₁₂ désigne hydrogène ou HSO₃ ;
   ou bien un groupe de formule suivante : 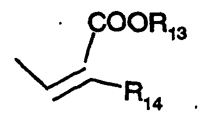 ou bien un groupe de formule suivante : 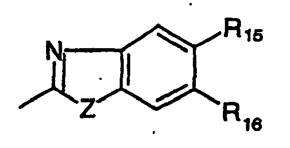 dans lesquelles:
   - Z désigne un atome d'oxygène ou un radical -NH- ;
   - R₁₃, R₁₄, R₁₅ et R₁₆, identiques ou différents, désignent un hydrogène, un halogène, un radical OH, un radical alkyle, saturé ou non, linéaire ou ramifié en C₁-C₁₀; un radical alcoxy, saturé ou non, linéaire ou ramifié en C₁-C₁₀ ou un groupe HSO₃

   On peut citer en particulier comme composé de formule (IV), l'acide benzène 1,4 [di(3-méthylidènecampho-10-sulfonique)] éventuellement sous forme partiellement ou totalement neutralisée qui répond à la formule (V) suivante : 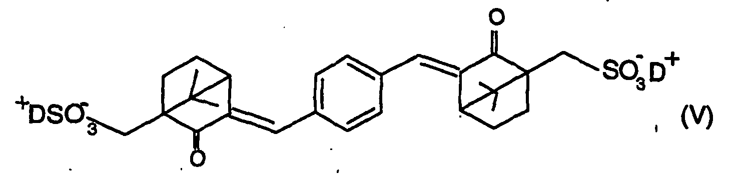 dans laquelle D désigne un atome d'hydrogène, un métal alcalin ou encore un radical NH(R₂₅)₃⁺ dans lequel les radicaux R₂₅, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺, et en particulier l'acide benzène 1,4-di(3-méthylidène-10-campho-sulfonique) commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL. M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HEUOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels de structure suivante (VI) : 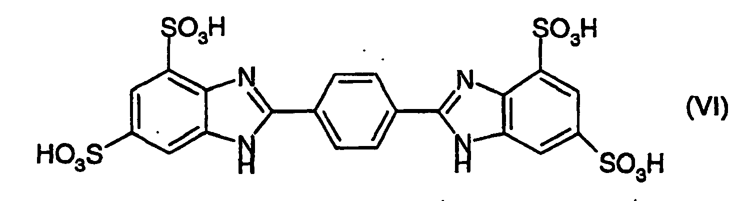 commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-1H-benzimidazol-2-yl]-benzoxazole;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy,1,1'-bis-(3-triméthylsilanyl-propyl)-1H,1'H-[2,2']bibenzimidazolyl-benzoxazole ;
   - le 2-[1-(3-trimethylsilanyl-propyl)-1H-benzimidazol-2-yl]-benzothiazole ; qui sont décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-methylènebis-[6-(2H benzotriazol-2-yl)4-(1,1,3,3-tétraméthylbutyl)phénol] commercialisé par la société Ciba Géigy sous la dénomination TINOSORB M ;
(9) leurs mélanges.

On peut aussi utiliser un mélange de plusieurs de ces filtres et un mélange de filtres UVB et de filtres UVA.

La composition selon l'invention trouve son application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

La composition selon l'invention peut par exemple être utilisée comme produit de soin et/ou de protection solaire pour le visage et/ou le corps sous forme de crèmes ou de laits.

Aussi, l'invention a encore pour objet l'utilisation cosmétique de la composition telle que définie ci-dessus pour le soin et/ou de protection solaire de la peau, des lèvres et/ou des cheveux, et/ou pour le maquillage de la peau et/ou des lèvres.

L'invention a aussi pour objet un procédé de traitement cosmétique pour protéger la peau, y compris le cuir chevelu, les cheveux, et/ou les lèvres contre le rayonnement solaire, caractérisé en ce qu'il consiste à appliquer sur la peau, les cheveux et/ou les lèvres, une composition telle que définie ci-dessus.

Les exemples ci-après d'émulsions selon l'invention sont donnés à titre d'illustration et sans caractère limitatif. Les quantités y sont données en % en poids, sauf mention contraire.

### Exemple 1 : Emulsion E/H de SPF 30

### Phase huileuse :

- Polyisobutylène à terminaison succinique esterifiée, sel de diéthyléthanolamine (Lubrizol 5603) 3 %
- Cyclohexadiméthylsiloxane 20 %
- Isohexadécane 30 %
- Dioxyde de titane enrobé PDMS
   (UV Titan X170 de la société Kemira) 10 % (en M.A.)

### Phase aqueuse

- Conservateurs qs %
- Mexoryl SX (filtre hydrosoluble) 3 %
- Eau déminéralisée qsp 100 %
   Mode opératoire : on homogénéise chacune des deux phases puis on les mélange sous agitation en dispersant de la phase aqueuse dans la phase huileuse.
   On obtient un lait fluide très doux, ne blanchissant pas à l'application. Son aspect au microscope est fin et régulier et on observe une bonne dispersion des pigments.
   Cette émulsion reste stable après deux mois de conservation à 45°C. Elle peut être utilisée comme soin quotidien protecteur et comme crème solaire pour le visage et le corps.

### Exemple 2 : Emulsion E/H de SPF 40

### Phase huileuse

- Polyisobutylène à terminaison succinique esterifiée, sel de diéthyléthanolamine (Lubrizol 5603) 3 %
- Cyclohexadiméthylsiloxane 30 %
- Isohexadécane 25 %
- Ethylhexylméthoxycinnamate 7 %
- Dioxyde de titane enrobé PDMS
   (UV Titan X170 de la société Kemira) 10 % (en M.A.)

### Phase aqueuse

- Conservateur qs
- Mexoryl SX (filtre hydrosoluble) 3 %
- Eau qsp 100 %

Mode opératoire: on homogénéise les deux phases puis on fait l'émulsion sous agitation par dispersion de la phase aqueuse dans la phase huileuse.

On obtient un lait fluide très doux, qui ne blanchit pas la peau lors de l'application. Son aspect au microscope est fin, régulier, on observe une bonne dispersion des pigments.

Cette émulsion reste stable après deux mois de conservation à 45°C. Elle peut être utilisée comme soin quotidien protecteur et comme crème solaire pour le visage et le corps.

### Exemple 3 : Emulsion E/H de SPF 20

### Phase huileuse

- Polyisobutylène à terminaison succinique esterifiée, sel de diéthyléthanolamine (Lubrizol 5603) 3 %
- Cyclohexadiméthylsiloxane 10 %
- lsohexadécane 25 %
- Ethythexylméthoxycinnamate 7 %

### Phase aqueuse

- Conservateur qs
- Mexoryl SX (filtre hydrosoluble) 3 %
- Nano-oxyde de titane à 30 % dans l'eau
   (MIRASUN TIW 60 de la société Rhodia) 10 %
- Eau qsp 100 %
   Mode opératoire : on homogénéise les deux phases, puis on fait l'émulsion sous agitation par dispersion de la phase aqueuse dans la phase huileuse.
   On obtient un lait fluide très doux, qui présente l'avantage de ne pas blanchir lors de l'application sur la peau. Son aspect au microscope est fin, régulier, on observe une bonne dispersion des pigments.
   Cette émulsion reste stable après deux mois de conservation à 45°C. Elle peut être utilisée comme soin quotidien protecteur et comme crème solaire pour le visage et le corps.

### Exemple 4 : Emulsion E/H de SPF 20

### Phase huileuse

- Polyisobutylène à terminaison succinique esterifiée, sel de diéthyléthanolamine (Lubrizol 5603) 3 %
- Cyclohexadécane 5 %
- Huile minérale 10 %
- Méthoxycinnamate de 2-éthylhexyle 7 %
- Benzophénone-3 2 %
- Oxyde de zinc enrobé par un polymère acrylique greffé silicone, dispersé dans cyclodimethylsiloxane
   (SPD Z1 de Shin-Etsu) 25 %

### Phase aqueuse

- Conservateur qs
- Sulfate de magnésium 1 %
- Eau qsp 100 %

Mode opératoire : on homogénéise les deux phases puis on fait l'émulsion sous agitation par dispersion de la phase aqueuse dans la phase huileuse.

On obtient un lait/crème souple et très doux, qui présente l'avantage de ne pas blanchir lors de l'application sur la peau. Son aspect au microscope est fin, régulier, on observe une bonne dispersion des pigments.

Cette émulsion reste stable après deux mois de conservation à 45°C. Elle peut être utilisée comme crème de jour.

## Revendications

1. Composition sous forme d'émulsion, contenant dans un milieu physiologiquement acceptable, au moins une phase aqueuse, au moins une phase huileuse, au moins un oxyde métallique sous forme de nanopigment dont la taille moyenne des particules est de 5 à 100 nm et au moins un oligomère ou un polymère dérivé de polyoléfine, comportant au moins une partie polaire.

2. Composition selon la revendication 1, **caractérisée en ce que** l'oligomère ou le polymère dérivé de polyoléfine comporte une partie apolaire polyoléfinique comprenant au moins 40 atomes de carbone et de préférence de 60 à 700 atomes de carbone.

3. Composition selon la revendication 2, **caractérisée en ce que** la partie apolaire polyoléfinique est choisie parmi les oligomères, les polymères et/ou les copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne.

4. Composition selon l'une quelconque des revendications précédentes, **caractérisée en ce que** l'oligomère ou le polymère dérivé de polyoléfine abaisse la tension interfaciale d'au moins 10 mN/m quand ledit oligomère ou polymère est présent à une concentration de 0,01% en poids par rapport au poids de phase huileuse.

5. Composition selon l'une quelconque des revendications précédentes, **caractérisée en ce que** la partie polaire peut être anionique, cationique, non ionique, zwitterionique ou amphotère.

6. Composition selon l'une quelconque des revendications précédentes, **caractérisée en ce que** la partie polaire est constituée de polyalkylène glycols, de polyalkylène imines, d'acides ou de diacides carboxyliques, de leurs anhydrides ou dérivés, et leurs mélanges.

7. Composition selon l'une quelconque des revendications précédentes, **caractérisée en ce que** la partie polaire est choisie dans le groupe comprenant le polyoxyéthylène, l'acide ou l'anhydride succinique et leurs dérivés.

8. Composition selon l'une quelconque des revendications précédentes, **caractérisée en ce que** l'oligomère ou le polymère dérivé de polyoléfine est issu de la réaction entre un dérivé de polyoléfine et au moins un acide choisi dans le groupe comprenant l'acide maléique ; l'anhydride maléique ; l'acide fumarique ; l'acide itaconique ; l'acide citraconique ; l'acide mésaconique ; l'acide aconitique ; leurs dérivés et leurs mélanges.

9. Composition selon l'une quelconque des revendications précédentes, **caractérisée en ce que** l'oligomère ou polymère dérivé de polyoléfine est un polyisobutylène à terminaison succinique éventuellement modifiée.

10. Composition selon l'une quelconque des revendications précédentes, **caractérisée en ce que** l'oligomère ou polymère dérivé de polyoléfine est le produit de la réaction de l'anhydride maléique avec le polyisobutylène.

11. Composition selon l'une quelconque des revendications précédentes, **caractérisée en ce que** la quantité d'oligomère(s) ou polymère(s) dérivé(s) de polyoléfine va de 0,1 à 20 % en poids de matière active par rapport au poids total de la composition.

12. Composition selon l'une quelconque des revendications précédentes, **caractérisée en ce que** l'oxyde métallique est choisi parmi les oxydes de titane, de zinc, de fer, de zirconium, de cérium ou leurs mélanges.

13. Composition selon l'une quelconque des revendications précédentes, **caractérisée en ce que** l'oxyde métallique est un oxyde enrobé ayant subi un ou plusieurs traitements avec un ou plusieurs composés choisis parmi l'alumine, la silice, les dérivés de l'aluminium, les composés du silicium, les composés du sodium, les oxydes de fer, les esters de fer, les acides gras, les alcools gras et leurs dérivés, la lécithine, les cires, les polymères (méth)acryliques, les composés fluorés.

14. Composition selon l'une quelconque des revendications précédentes, **caractérisée en ce que** la quantité d'oxydes métalliques va de 0,5 à 30 % en poids de matière active et de préférence de 2 à 20 % en poids de matière active par rapport au poids total de la composition.

15. Composition selon l'une quelconque des revendications précédentes, **caractérisée en ce qu'**elle contient en outre au moins un filtre solaire.

16. Composition selon la revendication précédente, **caractérisée en ce que** la phase huileuse représente de 5 à 80 % en poids par rapport au poids total de la composition.

17. Composition selon l'une quelconque des revendications précédentes, **caractérisée en ce qu'**elle constitue une émulsion eau-dans-huile.

18. Composition selon l'une quelconque des revendications précédentes, **caractérisée en ce qu'**elle constitue un produit de soin ou de protection solaire pour le visage et/ou le corps.

19. Utilisation cosmétique de la composition selon l'une quelconque des revendications 1 à **18,** pour le soin et/ou de protection solaire de la peau, des lèvres et/ou des cheveux, et/ou pour le maquillage de la peau et/ou des lèvres.

20. Procédé de traitement cosmétique pour protéger la peau, les cheveux, et/ou les lèvres contre le rayonnement solaire, **caractérisé en ce qu'**il consiste à appliquer sur la peau, les cheveux et/ou les lèvres, une composition selon l'une quelconque des revendications 1 à 18.

21. Utilisation d'au moins un oligomère ou un polymère dérivé de polyoléfine, comportant au moins une partie polaire, pour stabiliser une émulsion contenant au moins un oxyde métallique sous forme de nanopigment dont la taille moyenne des particules est de 5 à 100 nm.

## Patentansprüche

1. Als Emulsion vorliegende Zusammensetzung, die in einem physiologisch akzeptablen Medium mindestens eine wässerige Phase, mindestens eine Ölphase, mindestens ein Metalloxid als Nanopigment, dessen mittlere Partikelgröße im Bereich von 5 bis 100 nm liegt, und mindestens ein Oligomer oder Polymer enthält, das von einem Polyolefin abgeleitet ist und mindestens einen polaren Bereich aufweist.

2. Zusammensetzung nach Anspruch 1, **dadurch gekennzeichnet, dass** das von einem Polyolefin abgeleitete Oligomer oder Polymer einen apolaren polyolefinischen Bereich mit mindestens 40 Kohlenstoffatomen und vorzugsweise 60 bis 700 Kohlenstoffatomen aufweist.

3. Zusammensetzung nach Anspruch 2, **dadurch gekennzeichnet, dass** der polyolefinische apolare Bereich unter den Oligomeren, Polymeren und/oder Copolymeren von Ethylen, Propylen, 1-Buten, Isobuten, 1-Penten, 2-Methyl-1-buten, 3-Methyl-1-buten, 1-Hexen, 1-Hepten, 1-Octen, 1-Decen, 1-Undecen, 1-Dodecen, 1-Tridecen, 1-Tetradecen, 1-Pentadecen, 1-Hexadecen, 1-Heptadecen und 1-Octadecen ausgewählt ist.

4. Zusammensetzung nach einem der vorhergehenden Ansprüche, **dadurch gekennzeichnet, dass** das von einem Polyolefin abgeleitete Oligomer oder Polymer die Grenzflächenspannung um mindestens 10 mN/m absenkt, wenn das Oligomer oder Polymer in einer Konzentration von 0,01 Gew.-%, bezogen auf das Gewicht der Ölphase, enthalten ist.

5. Zusammensetzung nach einem der vorhergehenden Ansprüche, **dadurch gekennzeichnet, dass** der polare Bereich anionisch, kationisch, nichtionisch, zwitterionisch oder amphoter sein kann.

6. Zusammensetzung nach einem der vorhergehenden Ansprüche, **dadurch gekennzeichnet, dass** der polare Bereich aus Polyalkylenglykolen, Polyalkyleniminen, Carbonsäuren oder Dicarbonsäuren, deren Anhydriden oder Derivaten und deren Gemischen besteht.

7. Zusammensetzung nach einem der vorhergehenden Ansprüche, **dadurch gekennzeichnet, dass** der polare Bereich unter Polyoxyethylen, Bernsteinsäure oder Bernsteinsäureanhydrid und deren Derivaten ausgewählt ist.

8. Zusammensetzung nach einem der vorhergehenden Ansprüche, **dadurch gekennzeichnet, dass** das von einem Polyolefin abgeleitete Oligomer oder Polymer bei der Reaktion eines Polyolefinderivats und mindestens einer Säure gebildet wird, die unter Maleinsäure; Maleinsäureanhydrid; Fumarsäure; Itaconsäure; Citraconsäure; Mesaconsäure; Aconitsäure; deren Derivaten und deren Gemischen ausgewählt ist.

9. Zusammensetzung nach einem der vorhergehenden Ansprüche, **dadurch gekennzeichnet, dass** das von einem Polyolefin abgeleitete Oligomer oder Polymer ein Polyisobutylen mit gegebenenfalls modifizierter endständiger Bernsteinsäuregruppe ist.

10. Zusammensetzung nach einem der vorhergehenden Ansprüche, **dadurch gekennzeichnet, dass** das von einem Polyolefin abgeleitete Oligomer oder Polymer das Reaktionsprodukt von Maleinsäureanhydrid und Polyisobutylen ist.

11. Zusammensetzung nach einem der vorhergehenden Ansprüche, **dadurch gekennzeichnet, dass** der Mengenanteil des oder der von einem Polyolefin abgeleiteten Oligomere oder Polymere im Bereich von 0,1 bis 20 Gew.-% wirksame Substanz, bezogen auf das Gesamtgewicht der Zusammensetzung, liegt.

12. Zusammensetzung nach einem der vorhergehenden Ansprüche, **dadurch gekennzeichnet, dass** das Metalloxid unter den Oxiden von Titan, Zink, Zirconium, Cer oder deren Gemischen ausgewählt ist.

13. Zusammensetzung nach einem der vorhergehenden Ansprüche, **dadurch gekennzeichnet, dass** das Metalloxid ein umhülltes Oxid ist, das mit einer oder mehreren Verbindungen, die unter Aluminiumoxid, Siliciumoxid, Aluminiumderivaten, Siliciumverbindungen, Natriumverbindungen, Eisenoxiden, Eisenestern, Fettsäuren, Fettalkoholen und deren Derivaten, Lecithin, Wachsen, (Meth)acrylpolymeren und fluorierten Verbindungen ausgewählt sind, ein- oder mehrmals behandelt wurde.

14. Zusammensetzung nach einem der vorhergehenden Ansprüche, **dadurch gekennzeichnet, dass** der Mengenanteil der Metalloxide im Bereich von 0,5 bis 30 Gew.-% wirksame Substanz und vorzugsweise 2 bis 20 Gew.-% wirksame Substanz, bezogen auf das Gesamtgewicht der Zusammensetzung liegt.

15. Zusammensetzung nach einem der vorhergehenden Ansprüche, **dadurch gekennzeichnet, dass** sie ferner mindestens ein Sonnenschutzfilter enthält.

16. Zusammensetzung nach dem vorhergehenden Anspruch, **dadurch gekennzeichnet, dass** die Ölphase 5 bis 80 Gew.-%, bezogen auf das Gesamtgewicht der Zusammensetzung, ausmacht.

17. Zusammensetzung nach einem der vorhergehenden Ansprüche, **dadurch gekennzeichnet, dass** es sich um eine Wasser-in-Öl-Emulsion handelt.

18. Zusammensetzung nach einem der vorhergehenden Ansprüche, **dadurch gekennzeichnet, dass** sie ein Produkt zur Pflege oder zum Sonnenschutz für das Gesicht und/oder den Körper ist.

19. Kosmetische Verwendung der Zusammensetzung nach einem der Ansprüche 1 bis 18 für die Pflege und/oder den Sonnenschutz der Haut, der Lippen und/ oder der Haare und/ oder zum Schminken der Haut und/oder der Lippen.

20. Verfahren zur kosmetischen Behandlung zum Schutz der Haut, der Haare und/oder der Lippen gegen Sonnenlicht, **dadurch gekennzeichnet, dass** es darin besteht, dass auf die Haut, die Haare und/oder die Lippen eine Zusammensetzung nach einem der Ansprüche 1 bis 18 aufgetragen wird.

21. Verwendung mindestens eines von einem Polyolefin abgeleiteten Oligomers oder Polymers, das mindestens einen polaren Bereich aufweist, zur Stabilisierung einer Emulsion, die mindestens ein Metalloxid in Form eines Nanopigments enthält, dessen mittlere Partikelgröße im Bereich von 5 bis 100 nm liegt.

## Claims

1. Composition in the form of an emulsion, containing, in a physiologically acceptable medium, at least one aqueous phase, at least one oily phase, at least one metal oxide in the form of a nanopigment, the mean particle size of which is from 5 to 100 nm, and at least one oligomer or one polymer derived from a polyolefin, comprising at least one polar part.

2. Composition according to Claim 1, **characterized in that** the oligomer or the polymer derived from a polyolefin contains a polyolefinic apolar part comprising at least 40 carbon atoms and preferably from 60 to 700 carbon atoms.

3. Composition according to Claim 2, **characterized in that** the polyolefinic apolar part is chosen from oligomers, polymers and/or copolymers of ethylene, propylene, 1-butene, isobutene, 1-pentene, 2-methyl-l-butene, 3-methyl-1-butene, 1-hexene, 1-heptene, 1-octene, 1-decene, 1-undecene, 1-dodecene, 1-tridecene, 1-tetradecene, 1-pentadecene, 1-hexadecene, 1-heptadecene and 1-octadecene.

4. Composition according to any one of the preceding claims, **characterized in that** the oligomer or polymer derived from a polyolefin reduces the interfacial tension by at least 10 mN/m when said oligomer or polymer is present at a concentration of 0.01% by weight relative to the weight of the oily phase.

5. Composition according to any one of the preceding claims, **characterized in that** the polar part may be anionic, cationic, nonionic, zwitterionic or amphoteric.

6. Composition according to any one of the preceding claims, **characterized in that** the polar part consists of polyalkylene glycols, of polyalkyleneimines, of carboxylic or dicarboxylic acids, of anhydrides thereof or of derivatives thereof, and mixtures thereof.

7. Composition according to any one of the preceding claims, **characterized in that** the polar part is chosen from the group comprising polyoxyethylene, succinic acid or anhydride and derivatives thereof.

8. Composition according to any one of the preceding claims, **characterized in that** the oligomer or the polymer derived from a polyolefin is derived from the reaction between a polyolefin derivative and at least one acid chosen from the group comprising maleic acid; maleic anhydride; fumaric acid; itaconic acid; citraconic acid; mesaconic acid; aconitic acid; derivatives thereof and mixtures thereof.

9. Composition according to any one of the preceding claims, **characterized in that** the oligomer or polymer derived from a polyolefin is a polyisobutylene with an optionally modified succinic ending.

10. Composition according to any one of the preceding claims, **characterized in that** the oligomer or polymer derived from a polyolefin is the product of the reaction of maleic anhydride with polyisobutylene.

11. Composition according to any one of the preceding claims, **characterized in that** the amount of oligomer(s) or polymer(s) derived from a polyolefin ranges from 0.1 to 20% by weight of active material relative to the total weight of the composition.

12. Composition according to any one of the preceding claims, **characterized in that** the metal oxide is chosen from titanium, zinc, iron, zirconium and cerium oxides or mixtures thereof.

13. Composition according to any one of the preceding claims, **characterized in that** the metal oxide is a coated oxide which has undergone one or more treatments with one or more compounds chosen from alumina, silica, aluminium derivatives, silicon compounds, sodium compounds, iron oxides, iron esters, fatty acids, fatty alcohols and derivatives thereof, lecithin, waxes, (meth)acrylic polymers and fluoro compounds.

14. Composition according to any one of the preceding claims, **characterized in that** the amount of metal oxides ranges from 0.5 to 30% by weight of active material and preferably from 2 to 20% by weight of active material relative to the total weight of the composition.

15. Composition according to any one of the preceding claims, **characterized in that** it also contains at least one sunscreen.

16. Composition according to the preceding claim, **characterized in that** the oily phase represents from 5% to 80% by weight relative to the total weight of the composition.

17. Composition according to any one of the preceding claims, **characterized in that** it constitutes a water-in-oil emulsion.

18. Composition according to any one of the preceding claims, **characterized in that** it constitutes an antisun care or protection product for the face and/or the body.

19. Cosmetic use of the composition according to any one of Claims 1 to 18, for the antisun care and/or protection of the skin, the lips and/or the hair, and/or for making up the skin and/or the lips.

20. Cosmetic treatment process for protecting the skin, the hair and/or the lips against solar radiation, **characterized in that** it consists in applying to the skin, the hair and/or the lips a composition according to any one of Claims 1 to 18.

21. Use of at least one oligomer or one polymer derived from a polyolefin, containing at least one polar part, to stabilize an emulsion containing at least one metal oxide in the form of a nanopigment, the mean particle size of which is from 5 to 100 nm.
